# EUROPEAN PATENT APPLICATION

(11) **EP 3 892 730 A1**
(43) Date of publication of application: **13.10.2021**
(21) Application number: 20168483.4
(22) Date of filing: 07.04.2020
(51) Int. Cl.: C12P 19/12, C12P 19/24, C12P 19/02, C12P 19/14, A23L 2/84, A23L 5/20, A23L 21/10, A23L 29/00, A23L 33/125, C13B 20/00

(54) **IN SITU PRODUCTION OF ISOMALTULOSE**

(71) Applicant: Evonik Operations GmbH, 45128 Essen (DE)
(72) Inventor: HELLMERS, Frank, 45657 Recklinghausen (DE); HÜLLER, Thomas, 45772 Marl (DE); ÖHRLEIN, Johannes, 40476 Düsseldorf (DE); WOLTER, Jan, 40489 Düsseldorf (DE)
(74) Representative: Evonik Patent Association

(57) **Abstract**

The present invention relates to an *in-situ* method of producing isomaltulose in a final food product, the method comprising:
(a) contacting an isolated sucrose isomerase (EC 5.4.99.11) with a starting food product comprising sucrose, wherein the sucrose isomerase catalyses the conversion of sucrose to isomaltulose, glucose, fructose and trehalulose directly in the final food product.

## Description

### FIELD OF THE INVENTION

The present invention relates to an *in situ* method of production of isomaltulose in a food product. In particular, the method comprises contacting an isolated sucrose isomerase with sucrose found in an initial food product to directly convert the sucrose to isomalulose in the final food product.

### BACKGROUND OF THE INVENTION

Sucrose also known as saccharose is a disaccharide of glucose and fructose with the general formula C₁₂H₂₂O₁₁. Sucrose is a pure carbohydrate which may be easily and rapidly absorbed into blood. Overconsumption of sucrose may have several negative health effects. For example, dental caries or tooth decay, increased risk for chronic disease, development of obesity and insulin resistance etc. Therefore, the consumption of sucrose has to be monitored and controlled. An alternative to sucrose is isomaltulose (α-D-glucopyranosyl-1,6-fructose). Isomaltulose is also a disaccharide of glucose and fructose but has a different arrangement compared to sucrose. Isomaltulose may be a good substitute for sucrose as it provides the same flavour without the negative health effects that sucrose results in.

Usually, isomerization of sucrose to isomaltulose is carried out enzymatically with isomaltulose synthases (sucrose glucosylmutases, sucrose isomerases EC 5.4.99.11). In particular, the sucrose is brought into contact with a microorganism expressing sucrose isomerase. It is common for the microorganism to be immobilised. The cells are then left to incubate with the sucrose for a suitable period of time until most of the sucrose is converted to an isomaltulose mixture. The isomaltulose mixture is then purified and then dried. This dried isomaltulose mixture may then be used as a sweetener in various foods and drinks. DE1049800, DE2217628, EP 28900, EP49472 and EP 91063 describe methods with immobilized bacterial cells for enzymatic conversion of sucrose to isomaltulose.

These isomerization processes may be carried out with live or dead cells, with immobilized or free cells. DE3133123 and EP0915986 describe for example methods of immobilization of the enzyme catalysts with calcium alginate or ion exchangers, and EP0001099 describes a method with free, live cells, which can produce isomaltulose in the course of fermentation.

In all these cases, isomaltulose had to be first separated and dried and then added in the dried form into the food and drinks for human consumption. These steps take a lot of time and costs a lot of money. Accordingly, there is a need in the art for a cheaper and more efficient may of introducing isomaltulose into food and drinks.

### DESCRIPTION OF THE INVENTION

The present invention attempts to solve the problems above by providing a means of producing isomaltulose directly in food that may be consumed by human beings. In particular, there is provided a method of producing isomaltulose from sucrose directly in a food product where the food product is incubated with isolated sucrose isomerase. This method is advantageous as it removes the steps of separating and drying the isomaltulose mixture. The isomaltulose is directly produced in the food that is to be consumed. This method thus allows for costs and time to be saved.

According to one aspect of the present invention, there is provided an *in-situ* method of producing isomaltulose in a final food product, the method comprising:
(a) contacting an isolated sucrose isomerase (EC 5.4.99.11) with a starting food product comprising sucrose, wherein the sucrose isomerase catalyses the conversion of sucrose to isomaltulose directly in the final food product.

This method has the added advantage that sucrose has a higher solubility in water compared to isomaltulose. Therefore, a desired amount of sucrose may be added to the starting food product using any conventional method (i.e. heating, stirring etc.) without any complications or without any extra equipment or method required. The isolated sucrose isomerase is then directly added to the starting food product and the sucrose will be converted to isomaltulose in the final food product. Addition of isomaltulose to the final food product is more complicated as isomaltulose has not only a lower solubility in water compared to sucrose but isomaltulose is only able to be present in a maximum solid content of about 10% to produce stable solutions. Any higher concentration of isomaltulose in solution, may lead to crystallization of isomaltulose. As sucrose is a less complicated to work with compared to isomaltulose, an *in situ* method of converting sucrose to isomaltulose directly in the food product, may save time and effort.

The term *"In situ"* refers to a process wherein sucrose isomerase is directly contacted with a food product.

The term "contacting" refers to directly exposing a food product to a sucrose isomerase.

The term "food product" refers to any food or beverage which is consumable and includes sucrose as the starting food product and includes isomaltulose, glucose, fructose and trehalulose in the final food product. The starting food product may be a food product that had no contact with sucrose isomerase beforehand and then undergoes a process of converting sucrose to isomaltulose in the present of sucrose isomerase. The starting food product is therefore not the product that a customer may find on a supermarket shelf and still needs to undergo processing before it reaches the hands of a customer. The final food product in comparison is the food product a customer may find on a supermarket shelf. In particular, the final food product may have a low concentration of sucrose and a higher concentration of isomaltulose, glucose, fructose and trehalulose in comparison.

The term "low sucrose concentration" or "reducing the sucrose concentration" refers to a concentration level of sucrose in a food product that is less than the concentration level of sucrose in a corresponding food product, which has not been contacted with sucrose isomerase according to any aspect of the present invention. In one example, a low sucrose concentration in the final food product may mean the complete removal of the sucrose from the starting food product.

In one example, the food product may be a beverage (e.g. a sweet beverage) or a sweetener such as a syrup. The beverages may include fruit juices such as, orange, apple, grapefruit, grape, pineapple, cranberry, lemon, prune and lime juices, sport drinks, milk drinks such as chocolate, banana, strawberry flavoured milk.

Examples of syrups include maple syrup, strawberry syrup, blueberry syrup, and boysenberry syrup.

According to any aspect of the present invention, the solid content of isomaltulose, glucose, fructose and trehalulose in the final food product may be in the range of 1 to 80% (wt/wt). In particular, the solid content of isomaltulose, glucose, fructose and trehalulose may be in the range of 1 to 70, 1 to 60, 1 to 50, 1 to 40, 1 to 35, 1 to 30, 5 to 80, 5 to 70, 5 to 60, 5 to 50, 5 to 40, 5 to 35, 5 to 30, 10 to 80, 10 to 70, 10 to 60, 10 to 50, 10 to 45, 10 to 40, 10 to 35, 10 to 30, of the final food product (wt/wt). In some examples, when the food product is a natural juice (e.g. a non-concentrated juice) the solid content of isomaltulose, glucose, fructose and trehalulose may be in the range from 0.1% to 15%, particularly, 0.5% to 10% and more particularly 0.5% to 5%. The presence of the by-products, glucose, fructose and trehalulose improves the stability of isomaltulose in solution so that it may not crystalize out of the solution. For example, a solid content of at least 40% of isomaltulose, trehalulose, glucose, and fructose in solution may be stable without resulting in crystallization of isomaltulose. This enables the preparation of pre-mixes, which are often used in the beverage industry.

The initial sucrose level in the starting food product may vary with the type of food product. In one example, the % sucrose (w/v) in the food product may be between 2% and 75%, particularly between 10% and 55%, between 25% and 55% and more particularly, between 30 and 45%.

The starting food product according to any aspect of the present invention, may also comprise an undetectable concentration of isomaltulose which may be less than 1% (e.g., between 0 to 1.0% and 0 to 0.5%). In some examples, the isomaltulose level in the final food product may be increased by at least 0.5%, 1%, 5%, 10%, 15%, 20%, 25%, 30%, 40%, 50%, 75%, 80%, 85%, 90%, 95%, 200%, 300% and greater as compared to the starting food product.

After step (a) is carried out, the final food product may comprise about 0.5%, 1%, 2%, 5% or 10% sucrose. In particular, the final food product according to any aspect of the present invention may include about 0.3% of sucrose. More in particular, the final food product may comprise less than about 0.15% of sucrose. All % values in the specification refer to solid weight content. The term 'about' as used herein refers to a variation within 20 percent. In particular, the term "about" as used herein refers to +/- 20%, more in particular, +/-10%, even more in particular, +/- 5% of a given measurement or value.

In some examples, the sucrose level in the food product may be reduced by at least 5%, 10%, 15 I0, 20%, 25%, 30%, 35%, 40%, 45%, 50%, 55%, 60%, 65%, 70%, 75%, 85%, 90%, and 95% as compared to the starting food product. In on example, the amount of sucrose may be reduced by at least 50%, and in another example, the amount of sucrose may be reduced by at least 75% as compared to the starting food product. In other examples, between 20-100% of the sucrose in the starting food product may be enzymatically converted to isomerase according to any aspect of the present invention. In some examples, at least 40%, at least 50%, at least 60%, and also at least 70% of the sucrose in the starting food-product may be converted to isomerase by the method according to any aspect of the invention.

In some examples, the final food product may have 70 % - 90 % (w/w) isomaltulose, 5 - 15 % trehalulose (w/w), 1-5 % glucose (w/w) and 1-5 % (w/w) fructose. In particular, the final food product may have 70-95, 75-95, 80-95, 85-95, 90-95, 75-90, 80-90, 85-90, 70-85, 75-85, 80-85, 70-80, 75-80% (w/w) isomaltulose, 1-20, 5-20, 10-20, 15-20, 1-15, 5-15, 10-15, 1-10, 5-10 % (w/w) trehalulose, 1, 2, 3, 4, 5% (w/w) glucose and/or fructose.

Immobilization of the enzyme may allow for the economic use of high enzyme dosage and eliminates or reduces the need for removal or inactivation of residual enzyme from the product. In one example, the sucrose isomerase may remain in the final food product.

In another example, the method according to any aspect of the present invention, may further comprise a step of:
(a) deactivating the sucrose isomerase in the final food product.

Deactivation of the enzyme may be carried out by heating the final food product, by pasteurization or by other known methods. Pasteurization treatment may be from 15 seconds to 60 minutes, 15 seconds to 30 minutes, 5 minutes to 25 minutes or 10 minutes to 20 minutes at a temperature of 60°C to 95°C and generally at a temperature of 65°C to 75°C.

The amount of sucrose isomerase used according to any aspect of the present invention may vary depending on several variables. These variables include, the food product used in the method according to any aspect of the present invention, the amount of isomaltulose to be produced, the treatment time, the amount of sucrose present in the starting food product, and other process conditions. One of skill in the art will readily be able to determine the amount of sucrose isomerase to be used in the method according to any aspect of the present invention.

Further as known in the art, enzyme dose and reaction time are inversely proportional, and it may be therefore useful to calculate the product of dose and reaction time as a measure of the degree of reaction. For example, two hours at a dose of one unit per gram of sucrose (dose time = 2 U- hrs/g) is about equal to one hour of reaction at a dose of 2 U/g (also 2 U ∼ hrs/g). Under some conditions, a low dose time may be required and under some other conditions, a high dose time may be required. For example, when the pH of the food product is acidic, the sucrose isomerase may be less active and a greater dose time may be required.

In particular, the sucrose isomerase may be contacted with the starting food product under suitable conditions for the formation of isomaltulose and the by-products. The isomaltulose producing reaction may proceed under conditions where the sucrose isomerase works optimally. For example, the method according to any aspect of the present invention may proceed under a large range of temperature conditions, and this may be a function of time. In some examples, the temperature range may be between -10°C to 95°C, -5°C to 90°C, 1°C to 80°C, 1 °C to 75°C, 1 °C to 70°C, 5°C to 65°C, 5°C to 60°C, 5°C to 55°C, 10°C to 50°C, 5°C to 40°C, and 10°C to 40°C. In particular, the method according to any aspect of the present invention may proceed under the temperature range of 15 to 35 °C.

In some examples, the method according to any aspect of the present invention may proceed under pH conditions in the range of pH 3.0 to 8.0, about pH 3.0 to 7.0, pH 3.0 to 6.0 and pH 3.5 to 6.0. In particular, the method according to any aspect of the present invention may proceed under the pH 4 to 7, particularly, pH 4.5 to 7.

In some examples, the contacting according to step (a) will proceed for as little as 1 minute and in other examples for as long as several days or weeks. In some examples the contacting according to step (a) will occur for 30 minutes to 48 hours. In some examples, the contacting according to step (a) may continue during the shipping and storage of the food product prior to consumption. In other examples, the sucrose is enzymatically converted to isomaltulose in 1 minute to 60 hours.

The sucrose isomerase used according to any aspect of the present invention may be isolated from an organism selected from the group consisting of *Agrobacterium radiobacter, Azotobacter vinelandii, Bemisia argentifoli, Erwinia rhapontici, E. carotovora, Klebsiella planticola, Klebsiella terrigena, Leuconostoc mesenteroides, Pantoea dispersa, Protaminobacter rubrum, Pseudomonas mesoacidophila, Pseudomonas mesoacidophila, Pseudomonas putida, Serratia plymuthica, S. marcesens, Thermus aquaticus, Thermus filiformis, Thermus rubis* and combinations thereof.

In one example, the sucrose isomerase used according to any aspect of the present invention may be at least one genetically modified organism that has been genetically modified to increase the expression relative to the wild type cell of at least one sucrose isomerase. In particular, the genetically modified organism may be *Pichia pastoris* (Pena, D.A.et al (2018) Metabolic Engineering, 50:2-15).

The method according to any aspect of the present invention may comprise an earlier step of (ai) contacting an isolated invertase with a starting food product comprising sucrose, wherein the invertase (EC.3.2.1.26) catalyses the conversion of sucrose to glucose and fructose.

The invertase used according to any aspect of the present invention is further described in Kulshrestha, S. et al. (2013), journal of pharmacy research, 7: 792-797. Once the desired concentration of glucose and/or fructose is reached, the enzymatic activity of invertase may be stopped by using heat. A skilled person would understand what the desired concentration of glucose and/or fructose may be depending on the sweetness that is required in the final food product. In one example, more than 50, 55, 60, 65, 70, 75, 80, 85, 90 or 95% of the sucrose is broken down to glucose and fructose in the presence of the invertase. The remaining sucrose may then be converted to isomaltulose by the use of the enzyme isomerase. The inclusion of this step according to any aspect of the present invention removes the step of adding glucose and fructose to the final food product. Although glucose and fructose may already be produced as by-products of step (a) according to any aspect of the present invention, presence of even more glucose and fructose in the final food product may improve the crystallization stability of the final food product even more whilst maintaining the sweetness of the final food product, without jeopardizing the taste of the final food product. In some examples, when the invertase is used, the amount of isomaltulose present in the final product is much smaller and the amount of glucose and fructose is much higher.

### BRIEF DESCRIPTION OF THE FIGURES

Figure 1 is a graph showing the concentration of fructose, glucose, saccharose, isomaltulose and trehalulose at different time points with ice coffee in the presence of Sucrose Isomerase.
Figure 2 is a graph showing the concentration of fructose, glucose, saccharose, isomaltulose and trehalulose at different time points with a yogurt drink in the presence of Sucrose Isomerase.

### EXAMPLES

The foregoing describes preferred embodiments, which, as will be understood by those skilled in the art, may be subject to variations or modifications in design, construction or operation without departing from the scope of the claims. These variations, for instance, are intended to be covered by the scope of the claims.

### Example 1

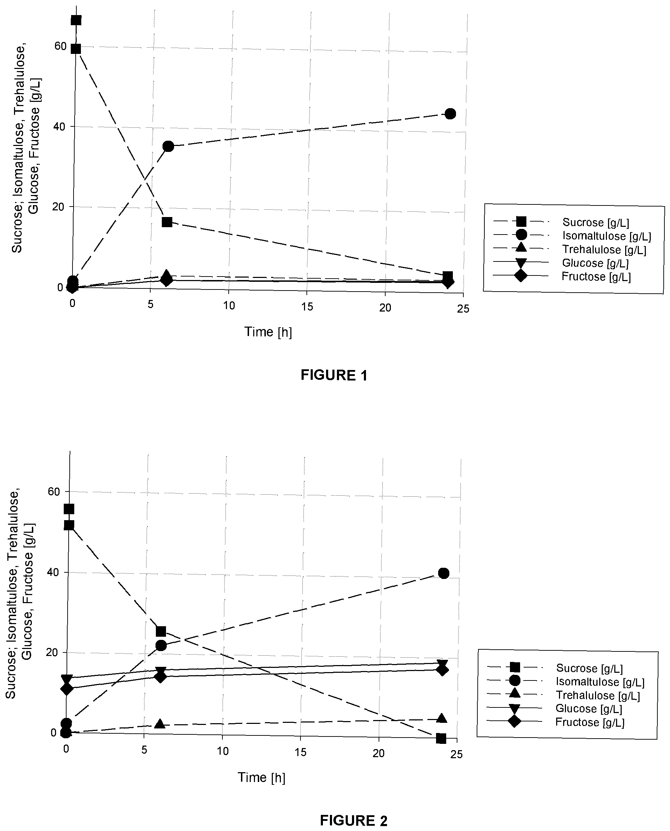

Ice coffee powder from the brand Nescafe was dissolved in water to make 10 ml of ice coffee in a 15 mL falcon tube. The pH value was not adjusted and was measured to be 5.02. Based on the information on the packaging, there was 88.4g of sugar for every 100g of coffee powder. In order to start the isomerization reaction 37.5 µL/mg of the enzyme sucrose isomerase (SI) was added. The reaction was allowed to take place at room temperature. Samples were taken at specific time points (0, 0.017, 6 and 24 hours) and the reaction was stopped by inactivating the enzyme for 3 minutes at 95°C in a thermomixer. A HPLC system was used to determine the concentration of fructose, glucose, saccharose, isomaltulose and trehalulose. The results are shown in Table 1 below and Figure 1.

**Table 1. Results of concentration of fructose, glucose, saccharose, isomaltulose and trehalulose at different time points with ice coffee.**

| **Time [h]** | **Sucrose [g/L]** | **Isomaltulose [g/L]** | **Trehalulose [g/L]** | **Glucose [g/L]** | **Fructose [g/L]** |
|---|---|---|---|---|---|
| 0 | 66,58 | 0,00 | 0,00 | 0,00 | 0,00 |
| 0,017 | 59,40 | 1,39 | 0,00 | 0,00 | 0,00 |
| 6 | 16,55 | 35,38 | 3,08 | 2,01 | 2,07 |
| 24 | 4,04 | 44,55 | 2,99 | 2,53 | 2,77 |

As can be seen from the results the enzyme activity of SI was calculated to be 10.83 µmol substrate min * mg enzyme and 7.66 µmol isomaltulose / min * mg enzyme.

Similarly, ice coffee powder was dissolved in 3.8% fat milk (i.e. 14g powder in 200ml of milk) with a pH of 6.1 and this resulted in the enzyme activity of SI being 14.41 µmol substrate min * mg enzyme and 8.77 µmol isomaltulose / min * mg enzyme.

### Example 2

A yogurt drink with 1.5% fat from the brand Berchtesgadener Land was tested in a similar manner as provided in Example 1. 10ml of the yogurt was placed in a 15 mL falcon tube and the pH value was not adjusted and was measured to be 4.4. Based on the information on the packaging, there was 10.7g of sugar for every 100g of yogurt. In order to start the isomerization reaction 37.5 µL/mg of the enzyme sucrose isomerase (SI) was added. The reaction was allowed to take place at room temperature. Samples were taken at specific time points (0, 0.017, 6 and 24 hours) and the reaction was stopped by inactivating the enzyme for 3 minutes at 95°C in a thermomixer. A HPLC system was used to determine the concentration of fructose, glucose, saccharose, isomaltulose and trehalulose. The results are shown in Table 2 below and Figure 2.

**Table 2. Results of concentration of fructose, glucose, saccharose, isomaltulose and trehalulose at different time points with drinking yogurt.**

| **Time [h]** | **Sucrose [g/L]** | **Isomaltulose [g/L]** | **Trehalulose [g/L]** | **Glucose [g/L]** | **Fructose [g/L]** |
|---|---|---|---|---|---|
| 0 | 55,71 | 0,00 | 0,00 | 13,33 | 10,89 |
| 0,017 | 51,67 | 2,27 | 0,00 | 13,61 | 11,00 |
| 6 | 25,64 | 22,04 | 2,24 | 15,93 | 14,29 |
| 24 | 0,00 | 41,06 | 4,67 | 18,70 | 17,07 |

As can be seen from the results the enzyme activity of SI was calculated to be 6.51 µmol substrate min * mg enzyme and 4.77 µmol isomaltulose / min * mg enzyme.

### Example 3

A chocolate milk drink from the brand Gut und Günstig was tested in a similar manner as provided in Example 1. 10ml of the chocolate milk was placed in a 15 mL falcon tube and the pH value was not adjusted and was measured to be 6.4. Based on the information on the packaging, there was 10.2g of sugar for every 100g of chocolate milk. In order to start the isomerization reaction 37.5 µL/mg of the enzyme sucrose isomerase (SI) was added. The reaction was allowed to take place at room temperature. Samples were taken at specific time points (0, 0.017, 6 and 24 hours) and the reaction was stopped by inactivating the enzyme for 3 minutes at 95°C in a thermomixer. A HPLC system was used to determine the concentration of fructose, glucose, saccharose, isomaltulose and trehalulose. The enzyme activity of SI was calculated to be 13.48 µmol substrate min * mg enzyme and 7.46 µmol isomaltulose / min * mg enzyme.

## Claims

1. An *in-situ* method of producing isomaltulose in a final food product, the method comprising:
(a) contacting an isolated sucrose isomerase (EC 5.4.99.11) with a starting food product comprising sucrose, wherein the sucrose isomerase catalyses the conversion of sucrose to isomaltulose, glucose, fructose and trehalulose directly in the final food product.

2. The method according to claim 1, wherein isomaltulose, glucose, fructose and trehalulose in the final food product is between 1 -35 % (w/w).

3. The method according to either claim 1 or 2, wherein the final food product comprises 75 % - 90 % (w/w) isomaltulose, 5 - 15 % (w/w) trehalulose, 1 - 5 % (w/w) glucose and 1 - 5 % (w/w) fructose.

4. The method according to any one of the preceding claims, wherein the method comprises a further step of:
(b) deactivating the sucrose isomerase in the final food product.

5. The method according to claim 4, wherein the deactivating of the sucrose isomerase is carried out by heating the final food product.

6. The method according to any one of the preceding claims, wherein the sucrose isomerase in step (a) is incubated with the starting food product at a pH selected from 4 to 7.

7. The method according to any one of the preceding claims, wherein the starting food product comprises an undetectable concentration of isomaltulose.

8. The method according to any one of the preceding claims, wherein the final food product comprises less than 0.3% of sucrose.

9. The method according to any one of the preceding claims, wherein the final food product comprises less than 0.15% of sucrose.

10. The method according to any one of the preceding claims, wherein at least 50% of the sucrose in the starting food product is converted to isomaltulose in the final food product.

11. The method according to any one of the preceding claims, wherein at least 75% of the sucrose in the starting food product is converted to isomaltulose in the final food product.

12. The method according to any one of the preceding claims, wherein the sucrose isomerase is isolated from an organism selected from the group consisting of *Agrobacterium radiobacter, Azotobacter vinelandii, Bemisia argentifoli, Erwinia rhapontici, E. carotovora, Klebsiella planticola, Klebsiella terrigena, Leuconostoc mesenteroides, Pantoea dispersa, Protaminobacter rubrum, Pseudomonas mesoacidophila, Pseudomonas mesoacidophila, Pseudomonas putida, Serratia plymuthica,* S. *marcesens, Thermus aquaticus, Thermus filiformis, Thermus rubis* and combinations thereof.

13. The method according to any one of the preceding claims, wherein the sucrose isomerase is isolated from at least one genetically modified organism that has been genetically modified to increase the expression relative to the wild type cell of at least one sucrose isomerase.

14. The method according to claim 13, wherein the genetically modified organism is selected from the group consisting of *Pichia pastoris* 15. The method according to any one of the preceding claims, comprising an earlier step of
(ai) contacting an isolated invertase (EC.3.2.1.26) with a starting food product comprising sucrose, wherein the invertase catalyses the conversion of sucrose to glucose and fructose.
